(19) Europäisches Patentamt
European Patent Office
Office européen des brevets

(11) **EP 2 303 109 B1**

(12) **EUROPEAN PATENT SPECIFICATION**

(45) Date of publication and mention
of the grant of the patent:
**14.09.2016 Bulletin 2016/37**

(51) Int Cl.:
***A61B 5/00*** *(2006.01)*

(21) Application number: **09772906.5**

(22) Date of filing: **29.06.2009**

(86) International application number:
**PCT/IB2009/006158**

(87) International publication number:
**WO 2010/001245 (07.01.2010 Gazette 2010/01)**

(54) **SYSTEMS AND METHOD FOR PROCESSING SIGNALS WITH REPETITIVE FEATURES**

SYSTEME UND VERFAHREN ZUR VERARBEITUNG VON SIGNALEN MIT SICH WIEDERHOLENDEN MERKMALEN

SYSTÈMES ET PROCÉDÉ DE TRAITEMENT DE SIGNAUX PRÉSENTANT DES ÉLÉMENTS RÉPÉTITIFS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.06.2008 US 76945**

(43) Date of publication of application:
**06.04.2011 Bulletin 2011/14**

(73) Proprietor: **Nellcor Puritan Bennett Ireland Mervue Galway (IE)**

(72) Inventors:
• **WATSON, James, Nicholas**
  **Fife KY11 8LE (GB)**
• **ADDISON, Paul, Stanley**
  **Edinburgh EH10 6UR (GB)**

(74) Representative: **Hargreaves, Timothy Edward et al Marks & Clerk LLP**
**Atholl Exchange**
**6 Canning Street**
**Edinburgh EH3 8EG (GB)**

(56) References cited:
EP-A- 1 611 847      WO-A-2008/065432
US-A1- 2006 258 921      US-B1- 6 409 659

**Description**

**Cross Reference To Related Applications**

This application claims priority to U.S. Provisional Application No. 61/076,945, entitled Systems and Methods For Processing Signals With Repetitive Features, filed on June 30,2008, the entirety of which is hereby incorporated herein by reference.

**Summary**

Aspects of the invention are in accordance with the appended claims. The present disclosure relates to signal processing systems and methods, and more particularly, to signal processing systems and methods for analyzing signals with repetitive components. The present disclosure may be used in connection with any signal having one or more repetitive components, including, for example, biosignals (e.g., a photoplethy sinograph (PPG) signal, electrocardiogram, electroencephalogram, electrogastrogram, electromyogram, heart rate signals, pathological sounds, ultrasound, or any other suitable biosignal), dynamic signals, non-destructive testing signals, condition monitoring signals, fluid signals, geophysical signals, astronomical signals, electrical signals, financial signals including financial indices, sound and speech signals, chemical signals, meteorological signals including climate signals, and/or any other suitable signal, and/or any combination thereof.

According to one aspect, the disclosure relates to a method for processing a signal, as defined in claim 1. The method includes receiving a signal having a repetitive component, for example, the pulse segments of a PPG signal. A plurality of features of the signal corresponding to the repetitive component are identified. Identification of the features may include, for example, identifying a plurality of turning points of the signal.

Segments of the signal, corresponding to the features, are transposed to form a stack of such segments. The start and end points of each segment are shared with respective adjacent segments. Information is then derived from analyzing the stack of segments.

According to the invention, transposing the plurality of segments of the signal to form a stack of segments includes aligning each subsequent segment next to the previous segment along a first axis. The length of each segment extends along a second axis that is perpendicular to the first axis. The amplitude of the each segment is represented in a third axis that is perpendicular to the first axis and the second axis. In one such embodiment, deriving the information includes detecting local maxima across either the first axis or the second axis of the stack to identify ridges. The ridges may then be analyzed to determine differential phase effects of respiration on a segment.

Additional suitable types of information which may be derived from PPG signals include blood pressure variation, changes in arterial compliance, and the severity of an illness. In various embodiments, deriving information includes identifying individual breaths by identifying local maxima in the stack of segments and/or identifying a respiration rate.

In certain embodiments, the method includes displaying the formed stack and/or the derived information on a display. In addition, or in the alternative, in certain embodiments, the method includes issuing an alert based on the derived information.

According to another aspect, the disclosure relates to computer readable media, which upon execution, causes out a processor to carry out the methods described above.

According to another aspect, the disclosure relates to a system for signal processing according to claim 8. The system includes a processor, and in some embodiments, a sensor, such as, without limitation, a pulse oximeter, and/or a display. The processor is capable of receiving a signal having at least one repetitive component and identifying a plurality of features of the signal corresponding to the at least one repetitive component. The processor is further capable of transposing segments of the signal to form a stack of segments. The segments have starting and end points adjacent the identified features. The processor is also capable of deriving information from the stack of segments.

A method and a system according to the preambles of claims 1 and 8 is known form US 6 409 659.

**Brief Description of the Drawings**

The patent or application file contains at least one drawing executed in color. Copies of this patent or patent application publication with color drawing(s) will be provided by the Office upon request and payment of the necessary fee.

The above and other features of the present disclosure, its nature and various advantages will be more apparent upon consideration of the following detailed description, taken in conjunction with the accompanying drawings in which:

**FIG. 1** shows an illustrative pulse oximetry system in accordance with an embodiment; **FIG. 2** is a block diagram of the illustrative pulse oximetry system of **FIG. 1** coupled to a patient in accordance with an embodiment; and

FIG. 3 is a block diagram of an illustrative signal processing system in accordance with some embodiments.

FIG. 4 is a graph of a photoplethysmograph signal a and filtered signal suitable for processing by the pulse oximetry systems of **FIG. 1** and **FIG. 2** and the signal processing system of **FIG. 3** according to an illustrative embodiment.

**FIGS. 5A-5C** are schematic depictions of a process of forming a stack of signal segments, according to an illustrative embodiment.

**FIG. 5D** is a flow chart of a method corresponding to the process depicted in **FIGS. 5A-5C.**

**FIGS. 6A** and **6B** are schematics of stacks of signal segments formed according to the process depicted in **FIGS. 5A-5C,** according to an illustrative embodiment.

**FIG. 7** is a schematic depicting the projection of a pulse signal onto a normalized baseline, according to an illustrative embodiment.

**FIG. 8** is a flow chart of a method of signal processing suitable for use by the pulse oximetry systems of **FIG. 1** and **FIG. 2** and the signal processing system of **FIG. 3,** according to an illustrative embodiment.

**FIG. 9** is a flow chart of a method of determining and outputting a breathing rate, according to an illustrative embodiment.

**FIG. 10** is a flow chart of a method of monitoring respiratory activity based on differential phase effects of breathing on pulse characteristics.

## Detailed Description

In medicine, a plethysmograph is an instrument that measures physiological parameters, such as variations in the size of an organ or body part, through an analysis of the blood passing through or present in the targeted body part, or a depiction of these variations. An oximeter is an instrument that may determine the oxygen saturation of the blood. One common type of oximeter is a pulse oximeter, which determines oxygen saturation by analysis of an optically sensed plethysmograph.

A pulse oximeter is a medical device that may indirectly measure the oxygen saturation of a patient's blood (as opposed to measuring oxygen saturation directly by analyzing a blood sample taken from the patient) and changes in blood volume in the skin. Ancillary to the blood oxygen saturation measurement, pulse oximeters may also be used to measure the pulse rate of the patient. Pulse oximeters typically measure and display various blood flow characteristics including, but not limited to, the oxygen saturation of hemoglobin in arterial blood.

An oximeter may include a light sensor that is placed at a site on a patient, typically a fingertip, toe, forehead or earlobe, or in the case of a neonate, across a foot. The oximeter may pass light using a light source through blood perfused tissue and photoelectrically sense the absorption of light in the tissue. For example, the oximeter may measure the intensity of light that is received at the light sensor as a function of time. A signal representing light intensity versus time or a mathematical manipulation of this signal (*e.g.*, a scaled version thereof, a log taken thereof, a scaled version of a log taken thereof, *etc.*) may be referred to as the photoplethysmograph (PPG) signal. In addition, the term "PPG signal," as used herein, may also refer to an absorption signal (*i.e.,* representing the amount of light absorbed by the tissue) or any suitable mathematical manipulation thereof. The light intensity or the amount of light absorbed may then be used to calculate the amount of the blood constituent (e.g., oxyhemoglobin) being measured as well as the pulse rate and when each individual pulse occurs.

The light passed through the tissue is selected to be of one or more wavelengths that are absorbed by the blood in an amount representative of the amount of the blood constituent present in the blood. The amount of light passed through the tissue varies in accordance with the changing amount of blood constituent in the tissue and the related light absorption. Red and infrared wavelengths may be used because it has been observed that highly oxygenated blood will absorb relatively less red light and more infrared light than blood with a lower oxygen saturation. By comparing the intensities of two wavelengths at different points in the pulse cycle, it is possible to estimate the blood oxygen saturation of hemoglobin in arterial blood.

When the measured blood parameter is the oxygen saturation of hemoglobin, a convenient starting point assumes a saturation calculation based on Lambert-Beer's law. The following notation will be used herein:

$$I(\lambda,t) = I_o(\lambda)\exp(-(s\beta_o(\lambda)+(1-s)\beta_r(\lambda))l(t)) \qquad (1)$$

where:

$\lambda$=wavelength;
t=time;
I=intensity of light detected;
$I_o$=intensity of light transmitted;
s=oxygen saturation;
$\beta_o$, $\beta_1$=empirically derived absorption coefficients; and
l(t)=a combination of concentration and path length from emitter to detector as a function of time.

The traditional approach measures light absorption at two wavelengths (e.g., red and infrared (IR)), and then calculates saturation by solving for the "ratio of ratios" as follows.

1. First, the natural logarithm of (1) is taken ("log" will be used to represent the natural logarithm) for IR and Red

$$\log I = \log I_o - (s\beta_o + (1-s)\beta_r)l \qquad (2)$$

2. (2) is then differentiated with respect to time

$$\frac{d\log I}{dt} = -(s\beta_o + (1-s)\beta_r)\frac{dl}{dt} \qquad (3)$$

3. Red (3) is divided by IR (3)

$$\frac{d\log I(\lambda_R)/dt}{d\log I(\lambda_{IR})/dt} \simeq \frac{s\beta_o(\lambda_R)+(1-s)\beta_r(\lambda_R)}{s\beta_o(\lambda_{IR})+(1-s)\beta_r(\lambda_{IR})} \qquad (4)$$

4. Solving for s

$$s = \frac{\dfrac{d\log I(\lambda_{IR})}{dt}\beta_r(\lambda_R) - \dfrac{d\log I(\lambda_R)}{dt}\beta_r(\lambda_{IR})}{\dfrac{d\log I(\lambda_R)}{dt}(\beta_o(\lambda_{IR})-\beta_r(\lambda_{IR})) - \dfrac{d\log I(\lambda_{IR})}{dt}(\beta_o(\lambda_R)-\beta_r(\lambda_R))}$$

Note in discrete time

$$\frac{d\log I(\lambda,t)}{dt} \simeq \log I(\lambda,t_2) - \log I(\lambda,t_1)$$

Using log A-log B=log A/B,

$$\frac{d \log I(\lambda,t)}{dt} \simeq \log\left(\frac{I(t_2,\lambda)}{I(t_1,\lambda)}\right)$$

So, (4) can be rewritten as

$$\frac{\dfrac{d \log I(\lambda_R)}{dt}}{\dfrac{d \log I(\lambda_{IR})}{dt}} \simeq \frac{\log\left(\dfrac{I(t_1,\lambda_R)}{I(t_2,\lambda_R)}\right)}{\log\left(\dfrac{I(t_1,\lambda_{IR})}{I(t_2,\lambda_{IR})}\right)} = R \qquad (5)$$

where **R** represents the "ratio of ratios." Solving (4) for s using (5) gives

$$s = \frac{\beta_r(\lambda_R) - R\beta_r(\lambda_{IR})}{R(\beta_o(\lambda_{IR}) - \beta_r(\lambda_{IR})) - \beta_o(\lambda_R) + \beta_r(\lambda_R)}.$$

From (5), **R** can be calculated using two points (e.g., PPG maximum and minimum), or a family of points. One method using a family of points uses a modified version of (5). Using the relationship

$$\frac{d \log I}{dt} = \frac{dI/dt}{I} \qquad (6)$$

now (5) becomes

$$\frac{\dfrac{d \log I(\lambda_R)}{dt}}{\dfrac{d \log I(\lambda_{IR})}{dt}} \simeq \frac{\dfrac{I(t_2,\lambda_R) - I(t_1,\lambda_R)}{I(t_1,\lambda_R)}}{\dfrac{I(t_2,\lambda_{IR}) - I(t_1,\lambda_{IR})}{I(t_1,\lambda_{IR})}}$$

$$= \frac{[I(t_2,\lambda_R) - I(t_1,\lambda_R)]I(t_1,\lambda_{IR})}{[I(t_2,\lambda_{IR}) - I(t_1,\lambda_{IR})]I(t_1,\lambda_R)}$$

$$= R \qquad (7)$$

which defines a cluster of points whose slope of y versus x will give **R** where

$$x(t) = [I(t_2,\lambda_{IR}) - I(t_1,\lambda_{IR})]I(t_1,\lambda_R)$$
$$y(t) = [I(t_2,\lambda_R) - I(t_1,\lambda_R)]I(t_1,\lambda_{IR})$$
$$y(t) = Rx(t) \qquad (8)$$

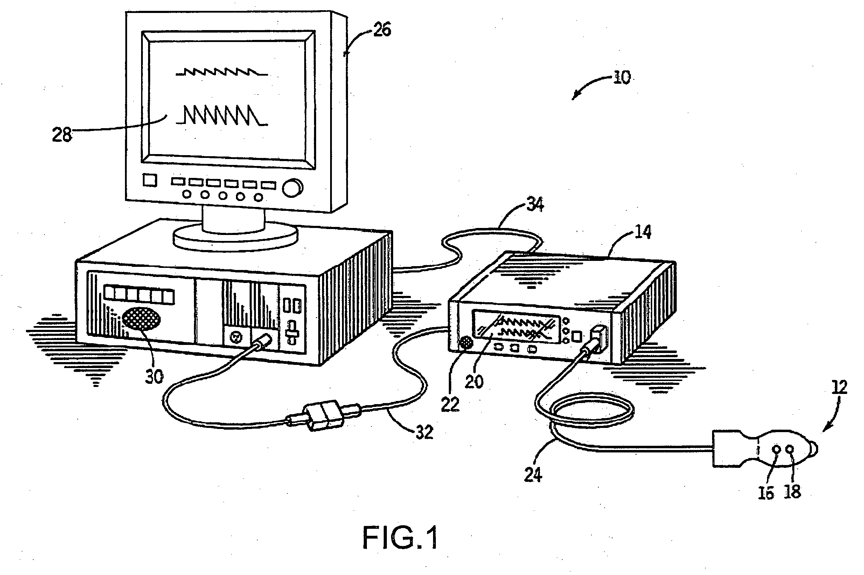

FIG. 1 is a perspective view of an embodiment of a pulse oximetry system **10**. System 10 may include a sensor **12** and a pulse oximetry monitor **14**. Sensor **12** may include an emitter **16** for emitting light at two or more wavelengths into a patient's tissue. A detector **18** may also be provided in sensor **12** for detecting the light originally from emitter **16** that emanates from the patient's tissue after passing through the tissue.

According to an embodiment, system **10** may include a plurality of sensors forming a sensor array in lieu of single sensor **12**. Each of the sensors of the sensor array may be a complementary metal oxide semiconductor (CMOS) sensor. Alternatively, each sensor of the array may be charged coupled device (CCD) sensor. In another embodiment, the sensor array may be made up of a combination of CMOS and CCD sensors. The CCD sensor may comprise a photoactive region and a transmission region for receiving and transmitting data whereas the CMOS sensor may be made up of an integrated circuit having an array of pixel sensors. Each pixel may have a photodetector and an active amplifier.

According to an embodiment, emitter **16** and detector **18** may be on opposite sides of a digit such as a finger or toe, in which case the light that is emanating from the tissue has passed completely through the digit. In an embodiment, emitter **16** and detector **18** may be arranged so that light from emitter **16** penetrates the tissue and is reflected by the tissue into detector **18**, such as a sensor designed to obtain pulse oximetry data from a patient's forehead.

In an embodiment, the sensor or sensor array may be connected to and draw its power from monitor **14** as shown. In another embodiment, the sensor may be wirelessly connected to monitor **14** and include its own battery or similar power supply (not shown). Monitor **14** may be configured to calculate physiological parameters based at least in part on data received from sensor **12** relating to light emission and detection. In an alternative embodiment, the calculations may be performed on the monitoring device itself and the result of the oximetry reading may be passed to monitor **14**. Further, monitor **14** may include a display **20** configured to display the physiological parameters or other information about the system. In the embodiment shown, monitor **14** may also include a speaker **22** to provide an audible sound that may be used in various other embodiments, such as for example, sounding an audible alarm in the event that a patient's physiological parameters are not within a predefined normal range.

In an embodiment, sensor **12**, or the sensor array, may be communicatively coupled to monitor **14** via a cable **24**. However, in other embodiments, a wireless transmission device (not shown) or the like may be used instead of or in addition to cable **24**.

In the illustrated embodiment, pulse oximetry system **10** may also include a multi-parameter patient monitor **26**. The monitor may be cathode ray tube type, a flat panel display (as shown) such as a liquid crystal display (LCD) or a plasma display, or any other type of monitor now known or later developed. Multi-parameter patient monitor **26** may be configured to calculate physiological parameters and to provide a display **28** for information from monitor **14** and from other medical monitoring devices or systems (not shown). For example, multiparameter patient monitor **26** may be configured to display an estimate of a patient's blood oxygen saturation generated by pulse oximetry monitor **14** (referred to as an "SpO$_2$" measurement), pulse rate information from monitor **14** and blood pressure from a blood pressure monitor (not shown) on display **28**.

Monitor **14** may be communicatively coupled to multi-parameter patient monitor **26** via a cable **32** or **34** that is coupled to a sensor input port or a digital communications port, respectively and/or may communicate wirelessly (not shown). In addition, monitor **14** and/or multi-parameter patient monitor **26** may be coupled to a network to enable the sharing of information with servers or other workstations (not shown). Monitor **14** may be powered by a battery (not shown) or by a conventional power source such as a wall outlet.

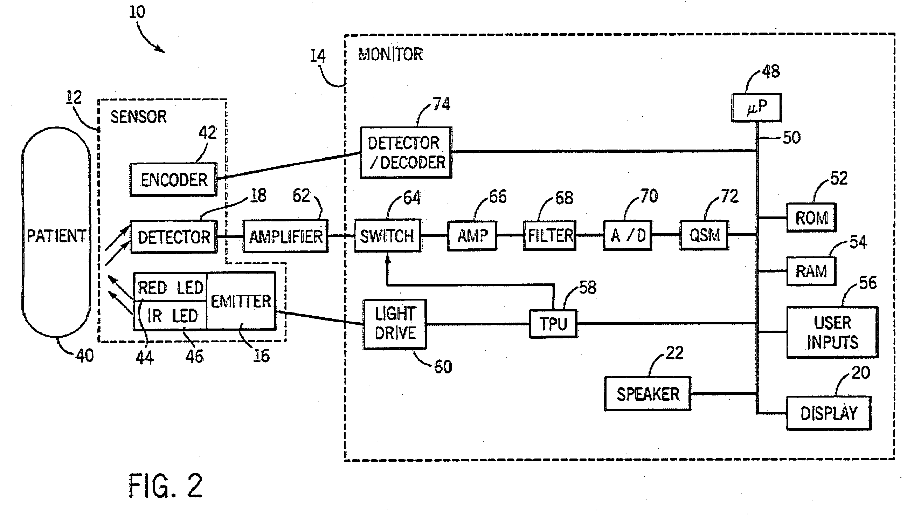

**FIG. 2** is a block diagram of a pulse oximetry system, such as pulse oximetry system **10** of **FIG. 1**, which may be coupled to a patient **40** in accordance with an embodiment. Certain illustrative components of sensor **12** and monitor **14** are illustrated in **FIG. 2**. Sensor **12** may include emitter **16**, detector **18**, and encoder **42**. In the embodiment shown, emitter **16** may be configured to emit at least two wavelengths of light (*e.g., RED* and *IR*) into a patient's tissue **40**. Hence, emitter **16** may include a *RED* light emitting light source such as *RED* light emitting diode (LED) **44** and an *IR* light emitting light source such as *IR* LED **46** for emitting light into the patient's tissue **40** at the wavelengths used to calculate the patient's physiological parameters. In one embodiment, the *RED* wavelength may be between about 600 nm and about 700 nm, and the *IR* wavelength may be between about 800 nm and about 1000 nm. In embodiments where a sensor array is used in place of single sensor, each sensor may be configured to emit a single wavelength. For example, a first sensor emits only a *RED* light while a second only emits an *IR* light.

It will be understood that, as used herein, the term "light" may refer to energy produced by radiative sources and may include one or more of ultrasound, radio, microwave, millimeter wave, infrared, visible, ultraviolet, gamma ray or X-ray electromagnetic radiation. As used herein, light may also include any wavelength within the radio, microwave, infrared, visible, ultraviolet, or X-ray spectra, and that any suitable wavelength of electromagnetic radiation may be appropriate for use with the present techniques. Detector **18** may be chosen to be specifically sensitive to the chosen targeted energy spectrum of the emitter **16**.

In an embodiment, detector **18** may be configured to detect the intensity of light at the *RED* and *IR* wavelengths. Alternatively, each sensor in the array may be configured to detect an intensity of a single wavelength. In operation, light may enter detector **18** after passing through the patient's tissue **40**. Detector **18** may convert the intensity of the received light into an electrical signal. The light intensity is directly related to the absorbance and/or reflectance of light in the tissue **40**. That is, when more light at a certain wavelength is absorbed or reflected, less light of that wavelength is received from the tissue by the detector **18**. After converting the received light to an electrical signal, detector **18** may send the signal to monitor **14,** where physiological parameters may be calculated based on the absorption of the *RED* and *IR* wavelengths in the patient's tissue **40**.

In an embodiment, encoder **42** may contain information about sensor **12**, such as what type of sensor it is (*e.g.,*

whether the sensor is intended for placement on a forehead or digit) and the wavelengths of light emitted by emitter **16.** This information may be used by monitor **14** to select appropriate algorithms, lookup tables and/or calibration coefficients stored in monitor **14** for calculating the patient's physiological parameters.

Encoder **42** may contain information specific to patient **40,** such as, for example, the patient's age, weight, and diagnosis. This information may allow monitor **14** to determine, for example, patient-specific threshold ranges in which the patient's physiological parameter measurements should fall and to enable or disable additional physiological parameter algorithms. Encoder **42** may, for instance, be a coded resistor which stores values corresponding to the type of sensor **12** or the type of each sensor in the sensor array, the wavelengths of light emitted by emitter **16** on each sensor of the sensor array, and/or the patient's characteristics. In another embodiment, encoder **42** may include a memory on which one or more of the following information may be stored for communication to monitor **14**: the type of the sensor **12**; the wavelengths of light emitted by emitter 16; the particular wavelength each sensor in the sensor array is monitoring; a signal threshold for each sensor in the sensor array; any other suitable information; or any combination thereof.

In an embodiment, signals from detector **18** and encoder **42** may be transmitted to monitor **14**. In the embodiment shown, monitor **14** may include a general-purpose microprocessor **48** connected to an internal bus **50**. Microprocessor **48** may be adapted to execute software, which may include an operating system and one or more applications, as part of performing the functions described herein. Also connected to bus **50** may be a read-only memory (ROM) **52**, a random access memory (RAM) **54**, user inputs **56**, display **20**, and speaker **22**.

RAM **54** and ROM **52** are illustrated by way of example, and not limitation. Any suitable computer-readable media may be used in the system for data storage. Computer-readable media are capable of storing information that can be interpreted by microprocessor **48**. This information may be data or may take the form of computer-executable instructions, such as software applications, that cause the microprocessor to perform certain functions and/or computer-implemented methods. Depending on the embodiment, such computer-readable media may include computer storage media and communication media. Computer storage media may include volatile and non-volatile, removable and non-removable media implemented in any method or technology for storage of information such as computer-readable instructions, data structures, program modules or other data. Computer storage media may include, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by components of the system.

In the embodiment shown, a time processing unit (TPU) **58** may provide timing control signals to a light drive circuitry **60,** which may control when emitter **16** is illuminated and multiplexed timing for the *RED* LED **44** and the *IR LED* **46**. TPU **58** may also control the gating-in of signals from detector **18** through an amplifier **62** and a switching circuit **64**. These signals are sampled at the proper time, depending upon which light source is illuminated. The received signal from detector **18** may be passed through an amplifier **66,** a low pass filter **68,** and an analog-to-digital converter **70**. The digital data may then be stored in a queued serial module (QSM) **72** (or buffer) for later downloading to RAM **54** as QSM **72** fills up. In one embodiment, there may be multiple separate parallel paths having amplifier **66,** filter **68,** and A/D converter **70** for multiple light wavelengths or spectra received.

In an embodiment, microprocessor **48** may determine the patient's physiological parameters, such as $SpO_2$ and pulse rate, using various algorithms and/or look-up tables based on the value of the received signals and/or data corresponding to the light received by detector **18**. Signals corresponding to information about patient **40,** and particularly about the intensity of light emanating from a patient's tissue over time, may be transmitted from encoder **42** to a decoder **74**. These signals may include, for example, encoded information relating to patient characteristics. Decoder **74** may translate these signals to enable the microprocessor to determine the thresholds based on algorithms or look-up tables stored in ROM **52**. User inputs **56** may be used to enter information about the patient, such as age, weight, height, diagnosis, medications, treatments, and so forth. In an embodiment, display **20** may exhibit a list of values which may generally apply to the patient, such as, for example, age ranges or medication families, which the user may select using user inputs **56**.

The optical signal through the tissue can be degraded by noise, among other sources. One source of noise is ambient light that reaches the light detector. Another source of noise is electromagnetic coupling from other electronic instruments. Movement of the patient also introduces noise and affects the signal. For example, the contact between the detector and the skin, or the emitter and the skin, can be temporarily disrupted when movement causes either to move away from the skin. In addition, because blood is a fluid, it responds differently than the surrounding tissue to inertial effects, thus resulting in momentary changes in volume at the point to which the oximeter probe is attached.

Noise (*e.g.*, from patient movement) can degrade a pulse oximetry signal relied upon by a physician, without the physician's awareness. This is especially true if the monitoring of the patient is remote, the motion is too small to be observed, or the doctor is watching the instrument or other parts of the patient, and not the sensor site. Processing pulse oximetry (*i.e.,* PPG) signals may involve operations that reduce the amount of noise present in the signals or otherwise identify noise components in order to prevent them from affecting measurements of physiological parameters derived from the PPG signals.

It will be understood that the present disclosure is applicable to any suitable signals and that PPG signals are used merely for illustrative purposes. Those skilled in the art will recognize that the present disclosure has wide applicability to other signals including, but not limited to other biosignals (*e.g.*, electrocardiogram, electroencephalogram, electrogastrogram, electromyogram, heart rate signals, pathological sounds, ultrasound, or any other suitable biosignal), dynamic signals, non-destructive testing signals, condition monitoring signals, fluid signals, geophysical signals, astronomical signals, electrical signals, financial signals including financial indices, sound and speech signals, chemical signals, meteorological signals including climate signals, and/or any other suitable signal, and/or any combination thereof.

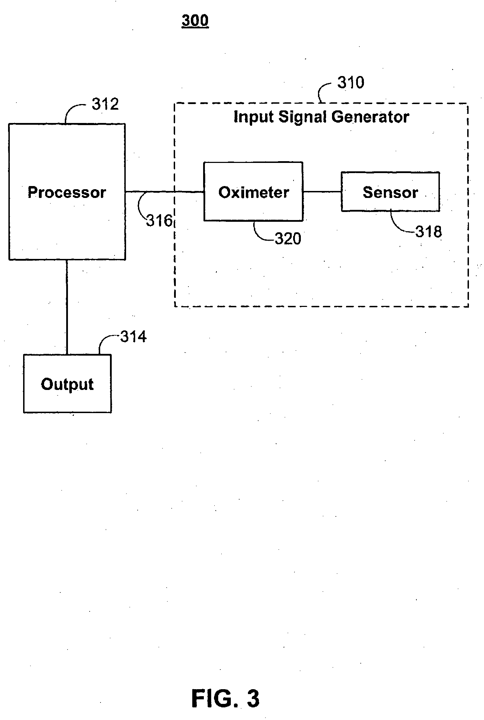

**FIG. 3** is an illustrative signal processing system in accordance with an embodiment. In this embodiment, input signal generator **310** generates an input signal **316**. As illustrated, input signal generator **310** may include oximeter **320** coupled to sensor **318**, which may provide as input signal **316**, a PPG signal. It will be understood that input signal generator **310** may include any suitable signal source, signal generating data, signal generating equipment, or any combination thereof to produce signal **316**. Signal **316** may be any suitable signal or signals, such as, for example, biosignals (*e.g.*, electrocardiogram, electroencephalogram, electrogastrogram, electromyogram, heart rate signals, pathological sounds, ultrasound, or any other suitable biosignal), dynamic signals, non-destructive testing signals, condition monitoring signals, fluid signals, geophysical signals, astronomical signals, electrical signals, financial signals including financial indices, sound and speech signals, chemical signals, meteorological signals including climate signals, and/or any other suitable signal, and/or any combination thereof.

In this embodiment, signal **316** may be coupled to processor **312**. Processor **312** may be any suitable software, firmware, and/or hardware, and/or combinations thereof for processing signal **316.** For example, processor **312** may include one or more hardware processors (*e.g.*, integrated circuits), one or more software modules, computer-readable media such as memory, firmware, or any combination thereof. Processor **312** may, for example, be a computer or may be one or more chips (*i.e.,* integrated circuits). Processor **312** may perform the calculations associated with the signal processing of the present disclosure as well as the calculations associated with any suitable interrogations of the processed signals. Processor **312** may perform any suitable signal processing of signal **316** to filter signal **316,** such as any suitable band-pass filtering, adaptive filtering, closed-loop filtering, and/or any other suitable filtering, and/or any combination thereof.

Processor **312** may be coupled to one or more memory devices (not shown) or incorporate one or more memory devices such as any suitable volatile memory device (*e.g.*, RAM, registers, *etc.*), non-volatile memory device (*e.g.*, ROM, EPROM, magnetic storage device, optical storage device, flash memory, *etc.*), or both. The memory may be used by processor **312** to, for example, store data corresponding to a continuous wavelet transform of input **signal 316,** such as data representing a scalogram. In one embodiment, data representing a scalogram may be stored in RAM or memory internal to processor **312** as any suitable three-dimensional data structure such as a three-dimensional array that represents the scalogram as energy levels in a time-scale plane. Any other suitable data structure may be used to store data representing a scalogram.

Processor **312** may be coupled to output **314**. Output **314** may be any suitable output device such as, for example, one or more medical devices (e.g., a medical monitor that displays various physiological parameters, a medical alarm, or any other suitable medical device that either displays physiological parameters or uses the output of processor **312** as an input), one or more display devices (*e.g.*, monitor, PDA, mobile phone, any other suitable display device, or any combination thereof), one or more audio devices, one or more memory devices (*e.g.*, hard disk drive, flash memory, RAM, optical disk, any other suitable memory device, or any combination thereof), one or more printing devices, any other suitable output device, or any combination thereof.

It will be understood that system **300** may be incorporated into system **10** (**FIGS. 1** and **2**) in which, for example, input signal generator **310** may be implemented as parts of sensor **12** and monitor **14** and processor **312** may be implemented as part of monitor **14.**

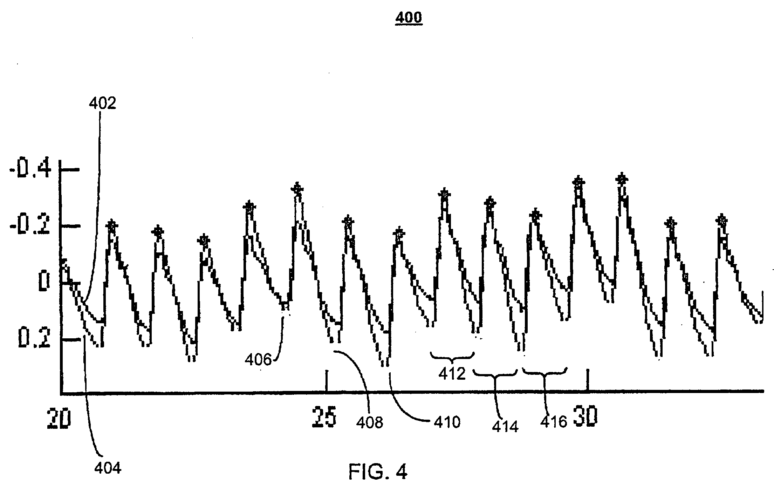

**FIG. 4** shows a graph of a PPG signal **402** and filtered PPG signal **404**, according to an embodiment. Pulse oximetry system **10** may process PPG signal **402** using any suitable filtering technique to create filtered PPG **404**. For example, pulse oximetry system **10** may use a band pass filtering technique to generate filtered PPG signal **404**. Pulse oximetry system **10** may use PPG signal **402** or filtered PPG signal **404** to identify features of the raw PPG signal (*i.e.,* PPG signal **402**). As illustrated in **FIG. 4,** pulse oximetry system **10** has filtered PPG signal **402** to more easily identify turning points (*e.g.*, turning points **406, 408, 410**) (i.e., points at which the first derivative of the signal changes from positive to negative or from negative to positive) associated with the heart rate. In one suitable approach, pulse oximetry system **10** may identify each turning point associated with a valley. In another suitable approach, pulse oximetry system **10** may identify each turning point associated with a peak or any other desired characteristic of PPG signal **402** such as a midpoint between a valley and peak or any other suitable location between the valleys and peaks. In another suitable implementation, particularly suited for analysis of signals which may include multiple peaks and valleys within a repetitive component, the pulse oximetry system **10** employs additional logic in addition to or instead of turning point detection to distinguish individual repetitive components. Such logic may include one or more of, for example, data indicating expected periodicity ranges for the signal, characteristic signal shape data, and pattern recognition logic. Such logic is configured to robustly distinguish individual repetitive components within the signal such that smaller signal variations within a repetitive component do not result in improper signal segmentation.

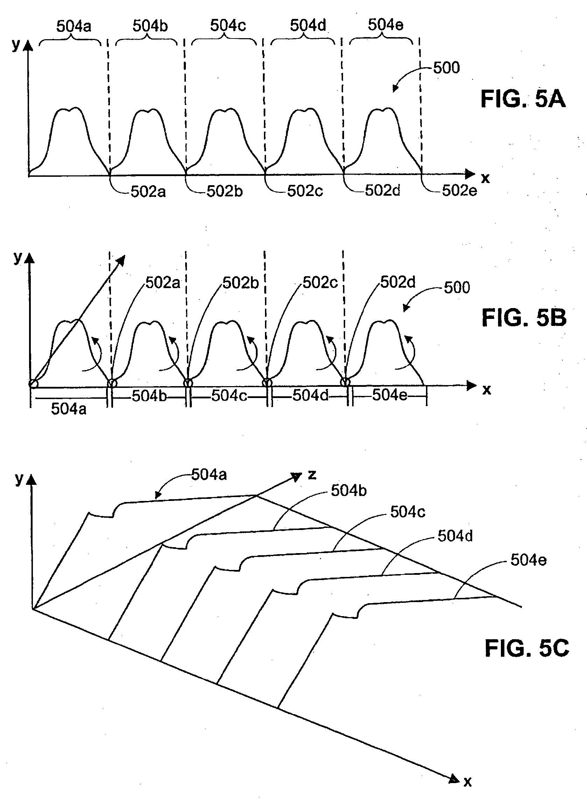

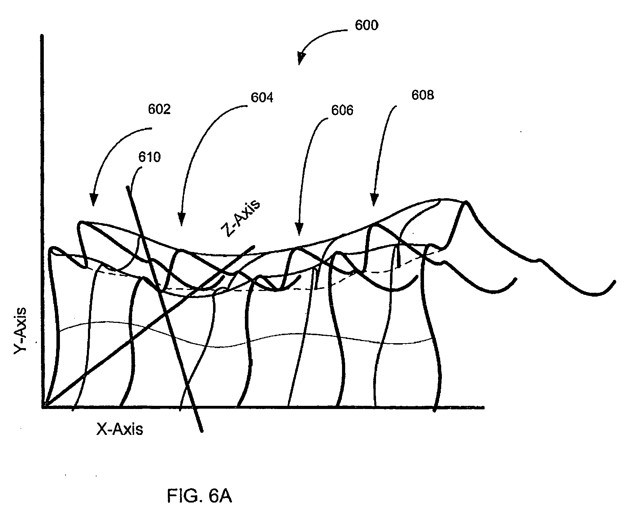

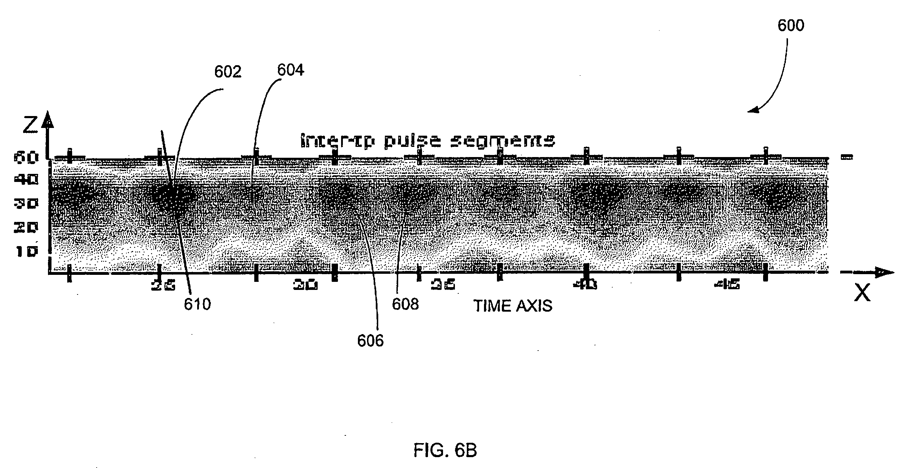

When the desired characteristics (which in this embodiment is the turning points associated with valleys) are identified, pulse oximetry system **10** may cut or break filtered PPG signal **404** (or alternatively, PPG signal **402**) at each identified feature (*e.g.*, each valley or turning point) to create a plurality of consecutive segments, such as segments **412, 414,** and **416,** corresponding to each heart pulse. If PPG signal **402** is being broken up, then pulse oximetry system **10** may break up PPG signal **402** at points corresponding in time to the identified features of filtered PPG signal **404.** Pulse oximetry system **10** may combine each pulse segment, *e.g.*, by interpolating between pulse segments, and stacking them against each other as shown in the schematic of **FIGS. 5A-5C**, resulting in the three dimensional renderings depicted in **FIGS. 6A** and **6B**.

Referring to **FIG. 4** and **FIGS. 5A-5C, FIG. 5A** shows an original signal **500** (*e.g.,* filtered PPG signal **404**) depicted in two dimensions X and Y. For illustrative purposes, **FIG. 5A** also depicts turning points **502a-502e** (generally "turning points **502**") to aid in discerning the beginning and end of segments **504a-504e** (generally "segments **504**").

**FIG. 5B** includes an additional axis (Z) added orthogonal to X and Y axes. In addition, **FIG. 5B** shows each individual segment **504a-504e** being rotated over the X-Z plane. After these rotations, the segments are then stacked as shown in **FIG. 5C.** In one embodiment a surface between the segments is derived by interpolation or other suitable estimation process.

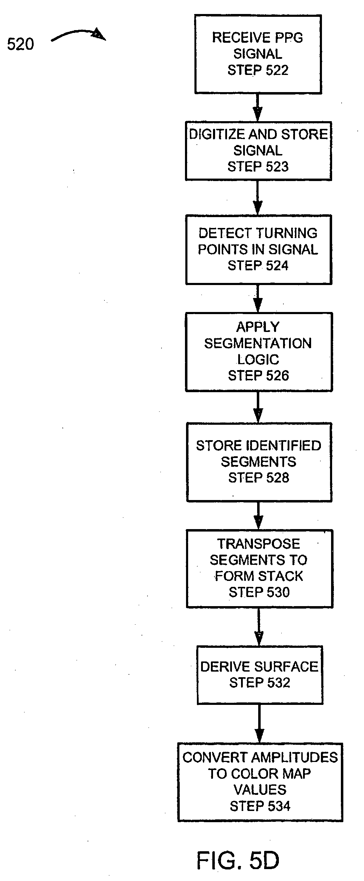

**FIG. 5D** is a flow chart of a method **520** for processing a PPG signal as depicted in **FIGS. 5A-5C.** The method **520** begins with the receipt of a PPG signal (**step 522**). A digital to analog converter digitizes the signal and stores it in memory (**step 523**). The memory may be any suitable form of random access memory.

As the signal is being received, individual heart beats are detected. Detecting heart beats includes detecting turning points in the signal (**step 524**), specifically turning points at which the slope of the signal switches from negative to positive, and applying segmentation logic (**step 526**) to determine whether the identified turning point represents the end of a heart beat. In one implementation , the segmentation logic includes a range of heart beat duration times. If the turning point occurs too close in time to the beginning of the heart beat, the turning point is ignored for segmentation purposes. In another implementation, the segmentation logic compares the amplitude of the signal at the detected turning point with the amplitude of the signal at the beginning of the segment. If the amplitude variation exceeds a predetermined threshold, the turning point is likewise ignored for segmentation purposes. In a further implementation, applying the segmentation logic includes applying a combination of a duration comparison and an amplitude comparison.

Upon detection of a turning point that meets the criteria for indicating the end of a segment, the detected segment is stored separately in memory (**step 528**). In one embodiment, the segment is stored as a bitmap of the signal in two dimensional space. In another embodiment, the signal is stored as a mathematical representation of the signal. Metadata indicating the position of the segment in the complete signal, along with date, time, and/or patient information may also be stored along with the segment.

As successive segments are stored, the segments are transposed to form the stack of segments (**step 530**). In one implementation, a pulse oximetry system **10** forms the stack of segments by forming a new image, having three dimensions, in which each segment is transposed adjacent a previous segment. In one example, as depicted in **FIGS. 5A-5C,** each segment is rotated 90 degrees about the Y axis such that it runs along the Z axis, with the Y axis still representing the magnitude of the signal. In another example, the length each segment remains parallel to the X axis and each successive segment is positioned behind or in front of a prior segment along the Z-axis. The distance between successive segments, in one implementation, corresponds to the length of the intervening segment. Alternatively, the segments may be spaced a common, arbitrary distance apart.

With the segments stacked in three dimensions, the pulse oximetry system 10 derives a surface joining the stacked segments **(step 532)**. In one embodiment, Y axis values for intervening points in the three-dimensional stack are interpolated based on the known signal values. Suitable interpolation techniques include, without limitation, linear interpolation, polynomial interpolation spline interpolation. Optionally, Y axis values from the resulting surface are converted to color values **(step 534)** according to a predetermined color map.

Pulse oximetry system **10** combines each pulse segment to create a three-dimensional surface as depicted in **FIG. 6A,** or, in one example, a two-dimensional surface coded according to a color map to represent the third axis, such as surface **600** illustrated in **FIG. 6B**. To generate surface 600, each pulse segment identified from filtered PPG signal **404** is stacked as shown in **FIG.5A-5C**, where each subsequent pulse segment may be stacked next to the previous (in time) pulse segment to create a series of pulse segments. If desired, the series of pulse segments may be interpolated to create a three- dimensional surface. Any suitable interpolation techniques may be used. Subsequent processing may be performed using a three-dimensional surface, a series of pulse segments, any other transposition, transformation, or interpolation of the series of pulse segments, or any combination thereof.

The X-axis of **FIGS. 6A** and **6B** is referred to as the "time-axis". According to embodiments, each point on the time-axis represents an entire pulse segment and thus has a time range associated with it. The X-axis for each pulse segment may be labeled based on any suitable time within the pulse segment such as the starting or end time of the segment or the time corresponding to the peak in each segment. (Note that the start location may also be modified to an integer reflecting the sequence of pulses, or any other timing or ordering that is appropriate to subsequent analysis). According to one embodiment, the Z-axis of **FIGS. 6A** and **6B** indicates each sequential data point within each pulse segment. As shown, each pulse segment starts on the X-axis and extends along the Z-axis. The pulse segments may vary in length. Therefore, pulse oximetry system **10** may equalize the lengths by, for example, padding the shorter lengths or may otherwise perform any suitable normalizing of pulse segment lengths.

The Y-axis represented **in FIG. 6A** is depicted by the height of the surface orthogonal to the X-Z plane. The Y-axis is represented in **FIG. 6B** as a color associated with a color map. The surface in **FIG. 6A** and the color map shown in **FIG. 6B** represent PPG signal **404** as a three-dimensional surface **600** as previously discussed. The three-dimensional surface may be analyzed by, for example, pulse oximetry system **10** to look for characteristics of individual pulses or similarities or differences between different pulses. This technique may be used, for example, to derive phase information, amplitude information, shape information, any other suitable information, or any combination thereof about pulses. This technique may also be used to analyze changes in the locations of characteristic features across a group of pulses.

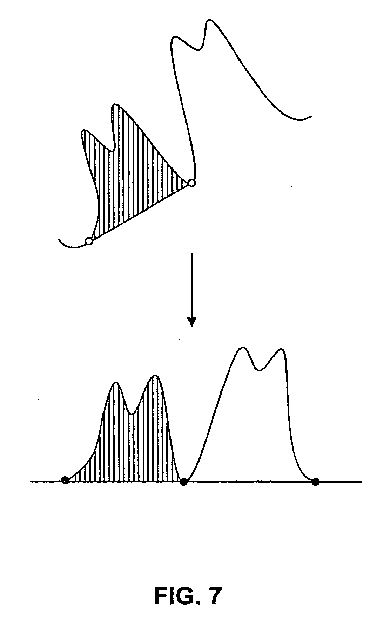

In alternative embodiments, the height profile of the original signal axis is the Y-coordinate of the plot (coming out of the page). In addition, the processing system may normalize the baseline of each pulse by projecting the pulse down onto a baseline as shown in **FIG. 7**. **FIG. 7** shows that the vertical heights taken from the pulse signal to the line drawn between endpoints are used to project the pulse onto a normalized baseline. Alternatively the baseline may be a best-fit curve, for example a cubic-spline curve fitted thorough the pulse endpoints, where again the heights from the signal to the baseline are taken and projected down onto a normalized baseline. The normalized baseline may be a horizontal baseline. In this way the original signal's baseline variations may be decoupled from amplitude variations of interest in the repeating pulse feature itself. This may be advantageous when, for example, large scale artifact features are present in the signal.

According to the invention the derived surface is used to determine respiration information (*e.g.*, individual breaths and respiration rate) by looking at local maxima. For example, local maxima **602**, **604**, **606**, and **608** are indicative of individual breaths. In other embodiments, ridges may be detected (*e.g.*, substantially vertical or horizontal ridges) to provide further information about the signal. For example, the skews of ridges from vertical or horizontal axes in **FIGS. 6A** and **6B**, such as skewed ridge **610,** may be used to detect the differential phase effect of respiration on the characteristic features within each pulse. This allows respiratory activity on each pulse component to be monitored individually. Hence differential activity within the pulse itself can be monitored or observed more easily. Other information that may be obtained from the derived surface may include long term and localized blood pressure variations. In addition pulse morphology changes with changes in arterial compliance, hence the method may be used to monitor compliance changes by determining the effect of compliance changes on the pulse characteristics. Further, pulse morphology may describe vascular response to severe illness including, but not limited to, sepsis and meningitis. Hence, the method detailed herein may be used to measure illness severity.

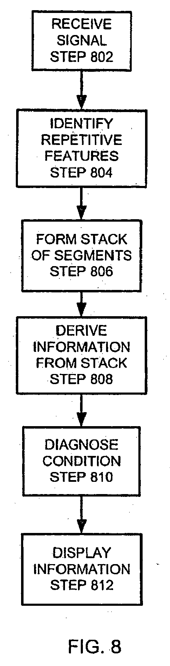

**FIG. 8** is a flow chart of a signal processing method **800** for analyzing a signal according to one illustrative embodiment. The signal processing method **800** begins with receiving a signal for processing (**step 802**). For example, the received signal may be a photoplethysmograph signal output by a pulse oximeter. The signal may have by a raw signal, or it may have been preprocessed. For example, the signal may have been filtered to remove noise or to isolate desired signal components. In alternative embodiments, the signal may be any signal having generally repetitive signal components, including, any of the signals disclosed above.

The signal processing method **800** continues with identifying a plurality of features of the signal corresponding to at least one repetitive component of the signal (**step 804**). Segments of the signal identified based on the features are then transposed to form a stack of segments (**step 806**). Each segment in the stack of segments has as start and end points portions of adjacent features.

Based on the stack, information about the signal is derived (**step 808**). In one embodiment, the information is derived automatically by a processor, for example, a processor incorporated into monitor **14** of **FIG.1**. In another embodiment, the information is derived by a processor external to pulse oximeter **10**. In still another embodiment, the information is derived by a practitioner visually evaluating the three dimensional surface **600***.* In addition, the derived information may optionally be further evaluated to diagnose the existence and/or severity of a condition (**step 810**) of a patient, system, or other object being monitored to obtain the signal. The derived information, including any diagnosis, may be displayed, for example, to a patient, researcher, supervisor, technician, or clinician (**step 812**).

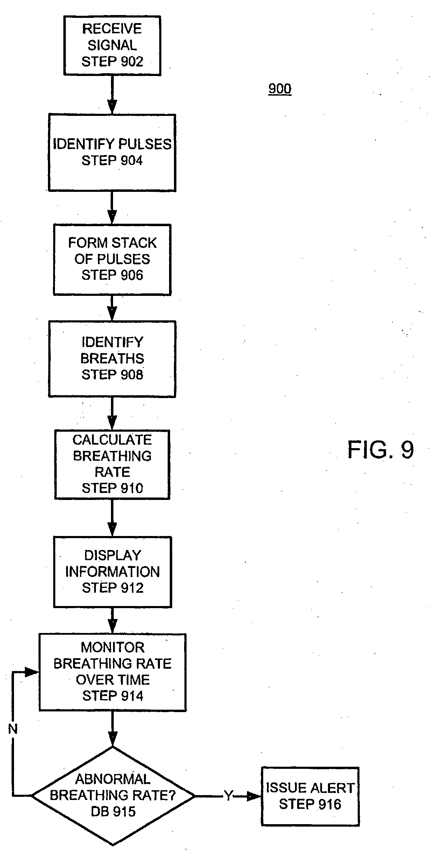

**FIG. 9** is a flow chart of a method **900** of determining and outputting a breathing rate, according to an illustrative example. As indicated above, analysis of the stack of segments yields a number of clinically valuable data values. For example, the stack of segments can be used to determine the breathing rate of a patient without having to monitor their respiration directly. Specifically, the amplitude of each segment, corresponding to the absorption of light modulated by volumetric changes in blood within its propagation path, varies based on its temporal relationship to a most recent breath. Local maxima in Y- dimension of the stack of segments correspond to individual breaths. Thus, calculating the frequency of such maxima yields a breathing rate.

The breathing rate detection method **900** begins with receipt of a PPG signal (**step 902**). Repetitive features are identified to detect individual signal segments corresponding to heart beat pulses (**step 904**). The pulse segments are then transposed with respect to one another as described above to form a stack of segments (**step 906**). As described above, the stack of segments may be in the form of a three-dimensional image, or alternatively, the Y-axis may be represented as a color map. Individual breaths are then detected by detecting and analyzing features, which may be characterized as local maxima, in the stack of segments (**step 908**) The features may be analyzed independently, or in conjunction with information obtained from a local scalogram, including, for example, its shape and and/or context. The frequency of such breaths is then calculated to determine a breathing rate (**step 910**) and the breathing rate and/or the stack of segments is displayed (**step 912**).

In one embodiment, the breathing rate detection method **900** also includes monitoring the breathing rate of a patient over time (**step 914**) and issuing alerts (**step 916**) upon detection of abnormal breathing rates (**DB 915**). Abnormality may be determined based on comparison of a current breathing rate to an individual's breathing rate history. For example, in one embodiment, an alert is issued (**step 916**) if a patient's breathing rate exceeds their average breathing rate by a predetermined multiple of, or by a predetermined number of standard deviations from, the patient's mean breathing rate. An alert may also be issued (**step 916**) if the patient's breathing falls below a similar threshold. In another embodiment, alerts are issued (**step 916**) based on a comparison of a patient's current breathing rate to fixed breathing rate thresholds. In each case, thresholds may be adjusted to take into account other physiological indications, for example to avoid false alerts during periods of sleep.

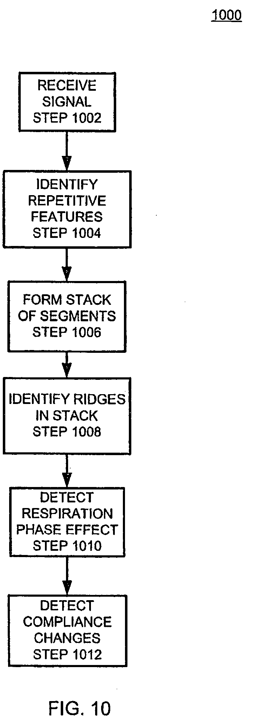

**FIG. 10** is a flow chart of a method **1000** of monitoring respiratory activity based on differential phase effects of breathing on pulse characteristics, according to an illustrative embodiment. As indicated above, analysis of the stack of segments can detect ridges primarily along both the X and Z axes. These ridges, however, may not be perfectly aligned with the axes. Deviation from axis alignment provides a measure of the differential phase effect of respiration on the characteristic features within each pulse, i.e., how the proximity of various features of the pulse varies in relation to its temporal proximity to a breath. For example it can indicate how the respiratory pressure variations are transmitted through the vascular system. Thus highlighting resistive hysterisis information useful in the monitoring of arterial compliance.

The method **1000** begins with the receipt of a PPG signal (**step 1002**). Repetitive features are identified (**step 1004**) to find signal segments. The signal segments are then stacked to form a stack of segments (**step 1006**). The stack of segments is analyzed to identify ridges, and their corresponding orientation (**step 1008**). Ridge shape and orientation are further analyzed to detect a respiratory differential phase affect (**step 1010**). These respiratory differential phase effects would be monitored to detect compliance changes (**step 1012**). Rapid compliance changes may be indicative of the efficacy of vasoconstrictive or vasodilative drugs administered to the patient. The detection of compliance changes may also be used in a continuous non-invasive blood pressure system as an indication that a recalibration is required.

### Claims

1. A method for processing a two-dimensional signal (402), comprising:

   receiving the two-dimensional signal (402) comprising a repetitive component;
   identifying a plurality of features of the two-dimensional signal (402) corresponding to the at least one repetitive component;
   transposing a plurality of segments (412) of the two-dimensional signal (402) to form a three-dimensional stack of segments (412), the segments having as starting and ending points adjacent identified features, wherein transposing the plurality of segments of the two-dimensional signal comprises aligning each subsequent segment of the plurality of segments next to each previous segment of the plurality of segments along a first axis;
   combining the plurality of segments in the three-dimensional stack of segments to create a three-dimensional surface, wherein the combining comprises interpolating the plurality of segments to create the three-dimensional surface; **characterized by**
   analyzing the three-dimensional surface to determine respiration information based on local maxima of the surface.

2. The method of claim 1, wherein the two-dimensional signal comprises a photoplethysmograph (PPG) signal.

3. The method of claim 2, wherein deriving information comprises identifying individual breaths in the three-dimensional surface, or deriving a respiration rate, or wherein the repetitive component comprises a pulse component of the PPG signal.

4. The method of claim 1, wherein identifying a plurality of features of the two-dimensional signal (402) comprises identifying a plurality of turning points (406) of the two-dimensional signal (402).

5. The method of claim 1,, wherein the length of each segment (412) extends along a second axis perpendicular to the first axis.

6. The method of claim 5, further comprising detecting local maxima (602) across either the first axis and/or the second axis to identify ridges, wherein
optionally, the two-dimensional signal comprises a PPG signal, and wherein deriving information comprises analyzing the ridges detected along the first axis to calculate the differential phase effect of respiration within each segment (412).

7. The method of claim 1 wherein the two-dimensional signal comprises a PPG signal and wherein deriving information comprises determining variations in blood pressure, or determining arterial compliance change, or determining illness severity.

8. A system for signal processing comprising:

a processor (312) capable of:

receiving a two-dimensional signal (402) comprising a repetitive component,
identifying a feature of the two-dimensional signal (402) corresponding to the repetitive component,
transposing a plurality of segments (412) of the two-dimensional signal to form a three-dimensional stack of segments (412), the segments comprising starting and ending points generally adjacent the identified feature, wherein transposing the plurality of segments of the two-dimensional signal comprises aligning each subsequent segment of the plurality of segments next to each previous segment of the plurality of segments along a first axis;
combining the plurality of segments in the three-dimensional stack of segments to create a three-dimensional surface, wherein the combining comprises interpolating the plurality of segments to create the three-dimensional surface;

**characterized in that** the processor is capable of

analyzing the three-dimensional surface to determine respiration information based on local maxima of the surface.

9. The system of claim 8, comprising a sensor (12), and wherein the processor (312) receives the two-dimensional signal (402) from the sensor (12), and
optionally, the sensor (12) comprises a pulse oximeter (320)and the two-dimensional signal comprises a photoplethysmograph (PPG) signal.

10. The system of claim 9, wherein deriving information comprises identifying individual breaths by identifying local maxima (602) in the three-dimensional surface, or deriving a respiration rate, or wherein the repetitive component is a pulse component of the PPG signal.

11. The system of claim 8, wherein identifying a feature of the two-dimensional signal (402) comprises identifying a turning point (406) of the two-dimensional signal (402), and/or wherein the processor (312) is capable of issuing an alert based in part on the derived information.

12. The system of claim 8, wherein the length of each segment (412) of the plurality of segments extends along a second axis perpendicular to the first axis.

13. The system of claim 11, wherein the processor (312) is capable of identifying ridges in the stack of the plurality of segments, and
optionally, the system comprises a pulse oximeter (320), wherein the two-dimensional signal comprises a photoplethysmograph (PPG) signal and deriving information comprises analyzing the ridges detected along the first axis to calculate the differential phase effect of respiration within each segment (412) of the plurality of segments.

**14.** The system of claim 13, comprising a pulse oximeter, wherein the two-dimensional signal comprises a photoplethysmograph (PPG) signal and deriving information comprises determining variations in blood pressure, or determining arterial compliance change, or determining illness severity.

**15.** A computer readable medium storing computer readable instructions, which, when executed by a processor (312), cause the processor (312) to a carry out a method according to any of claims 1 to 7.

**Patentansprüche**

**1.** Verfahren zum Verarbeiten eines zweidimensionalen Signals (402), umfassend:

Empfangen des zweidimensionalen Signals (402), das eine sich wiederholende Komponente umfasst;
Identifizieren einer Vielzahl von Merkmalen des zweidimensionalen Signals (402), die der mindestens einen sich wiederholenden Komponente entsprechen;
Umgruppieren einer Vielzahl von Segmenten (412) des zweidimensionalen Signals (402), um einen dreidimensionalen Stapel von Segmenten (412) zu bilden, wobei die Segmente benachbarte identifizierte Merkmale als Anfangs- und Endpunkte haben, worin das Umgruppieren der Vielzahl von Segmenten des zweidimensionalen Signals umfasst: Ausrichten jedes nachfolgenden Segments der Vielzahl von Segmenten als Nächstes zu jedem vorhergehenden Segment der Vielzahl von Segmenten entlang einer ersten Achse;
Kombinieren der Vielzahl von Segmenten in dem dreidimensionalen Stapel von Segmenten, um eine dreidimensionale Oberfläche zu bilden, worin das Kombinieren umfasst: Interpolieren der Vielzahl von Segmenten, um die dreidimensionale Oberfläche zu erzeugen; **gekennzeichnet durch**:

Analysieren der dreidimensionalen Oberfläche, um Atmungsinformation auf der Grundlage von lokalen Maxima der Oberfläche zu bestimmen.

**2.** Verfahren nach Anspruch 1, worin das zweidimensionale Signal ein Fotoplethysmograf-(PPG-)Signal umfasst.

**3.** Verfahren nach Anspruch 2, worin das Ableiten von Information das Identifizieren einzelner Atemzüge in der dreidimensionalen Oberfläche oder das Ableiten einer Atmungsrate umfasst oder worin die sich wiederholende Komponente eine Pulskomponente des PPG-Signals umfasst.

**4.** Verfahren nach Anspruch 1, worin das Identifizieren einer Vielzahl von Merkmalen des zweidimensionalen Signals (402) umfasst: Identifizieren einer Vielzahl von Wendepunkten (406) des zweidimensionalen Signals (402).

**5.** Verfahren nach Anspruch 1, worin die Länge jedes Segments (412) sich entlang einer zweiten Achse erstreckt, die senkrecht zu der ersten Achse ist.

**6.** Verfahren nach Anspruch 5, ferner umfassend: Ermitteln lokaler Maxima (602) über der ersten Achse und/oder der zweiten Achse, um Grate zu identifizieren, worin das zweidimensionale Signal optional ein PPG-Signal umfasst und worin das Ableiten von Information umfasst: Analysieren der entlang der ersten Achse ermittelten Grate, um den differenziellen Phaseneffekt der Atmung innerhalb jedes Segments (412) zu berechnen.

**7.** Verfahren nach Anspruch 1, worin das zweidimensionale Signal ein PPG-Signal umfasst und worin das Ableiten von Information umfasst: Bestimmen von Blutdruckschwankungen oder Bestimmen der Veränderung der Arterien-Compliance oder Bestimmen der Krankheitsschwere.

**8.** System zur Signalverarbeitung, umfassend:

einen Prozessor (312), der imstande ist zum:

Empfangen eines zweidimensionalen Signals (402), das eine sich wiederholende Komponente umfasst;
Identifizieren einer Vielzahl von Merkmalen des zweidimensionalen Signals (402), die der sich wiederholenden Komponente entsprechen;
Umgruppieren einer Vielzahl von Segmenten (412) des zweidimensionalen Signals (402), um einen dreidimensionalen Stapel von Segmenten (412) zu bilden, wobei die Segmente Anfangs- und Endpunkte umfassen, die im Allgemeinen dem identifizierten Merkmal benachbart sind, worin das Umgruppieren der Vielzahl von Segmenten des zweidimensionalen Signals umfasst: Ausrichten jedes nachfolgenden Segments der Vielzahl von Segmenten als Nächstes zu jedem vorhergehenden Segment der Vielzahl von Segmenten entlang einer ersten Achse;

Kombinieren der Vielzahl von Segmenten in dem dreidimensionalen Stapel von Segmenten, um eine dreidimensionale Oberfläche zu bilden, worin das Kombinieren umfasst: Interpolieren der Vielzahl von Segmenten, um die dreidimensionale Oberfläche zu erzeugen;

**dadurch gekennzeichnet, dass** der Prozessor imstande ist zum:

Analysieren der dreidimensionalen Oberfläche, um Atmungsinformation auf der Grundlage von lokalen Maxima der Oberfläche zu bestimmen.

9. System nach Anspruch 8, umfassend einen Sensor (12), und worin der Prozessor (312) das zweidimensionale Signal (402) von dem Sensor (12) empfängt, und

der Sensor (12) optional ein Pulsoxymeter (320) umfasst und das zweidimensionale Signal ein Fotoplethysmograf-(PPG-)Signal umfasst.

10. System nach Anspruch 9, worin das Ableiten von Information das Identifizieren einzelner Atemzüge durch Identifizieren lokaler Maxima (602) in der dreidimensionalen Oberfläche oder das Ableiten einer Atmungsrate umfasst oder worin die sich wiederholende Komponente eine Pulskomponente des PPG-Signals umfasst.

11. System nach Anspruch 8, worin das Identifizieren eines Merkmals des zweidimensionalen Signals (402) das Identifizieren eines Wendepunkts (406) des zweidimensionalen Signals (402) umfasst und/oder worin der Prozessor (312) zum Ausgeben eines Alarms imstande ist, der teilweise auf der abgeleiteten Information beruht.

12. System nach Anspruch 8, worin die Länge jedes Segments (412) der Vielzahl von Segmenten sich entlang einer zweiten Achse erstreckt, die senkrecht zu der ersten Achse ist.

13. System nach Anspruch 11, worin der Prozessor (312) zum Identifizieren von Graten in dem Stapel der Vielzahl von Segmenten imstande ist, und

das System optional ein Pulsoxymeter (320) umfasst, worin das zweidimensionale Signal ein Fotoplethysmograf-(PPG-)Signal umfasst und das Ableiten von Information umfasst: Analysieren der entlang der ersten Achse ermittelten Grate, um den differenziellen Phaseneffekt der Atmung innerhalb jedes Segments (412) der Vielzahl von Segmenten zu berechnen.

14. System nach Anspruch 13, ein Pulsoxymeter umfassend, worin das zweidimensionale Signal ein Fotoplethysmograf-(PPG-)Signal umfasst und das Ableiten von Information umfasst: Bestimmen von Blutdruckschwankungen oder Bestimmen der Veränderung der Arterien-Compliance oder Bestimmen der Krankheitsschwere.

15. Computerlesbares Medium, das computerlesbare Anweisungen speichert, die, wenn sie durch einen Prozessor (312) ausgeführt werden, den Prozessor (312) veranlassen, ein Verfahren nach einem der Ansprüche 1 bis 7 auszuführen.

**Revendications**

1. Procédé de traitement d'un signal bidimensionnel (402), comprenant les étapes suivantes :

recevoir le signal bidimensionnel (402) comprenant une composante répétitive ;

identifier une pluralité de caractéristiques du signal bidimensionnel (402) correspondant à l'au moins une composante répétitive ;

transposer une pluralité de segments (412) du signal bidimensionnel (402) pour former un empilement tridimensionnel de segments (412), les segments ayant comme points de début et de fin des caractéristiques identifiées adjacentes, où transposer la pluralité de segments du signal bidimensionnel comprend d'aligner chaque segment subséquent de la pluralité de segments à côté de chaque segment précédent de la pluralité de segments le long d'un premier axe ;

combiner la pluralité de segments dans l'empilement tridimensionnel de segments pour créer une surface tridimensionnelle, où la combinaison comprend d'interpoler la pluralité de segments pour créer la surface tridimensionnelle ; **caractérisé par** l'étape suivante :

analyser la surface tridimensionnelle pour déterminer des informations de respiration sur la base de maximas locaux de la surface.

2. Procédé selon la revendication 1, dans lequel le signal bidimensionnel comprend un signal de photopléthysmographe (PPG).

3. Procédé selon la revendication 2, dans lequel dériver des informations comprend d'identifier des respirations individuelles dans la surface tridimensionnelle, ou de dériver une vitesse de respiration, ou dans lequel la composante répétitive comprend une composante d'impulsion du signal de PPG.

4. Procédé selon la revendication 1, dans lequel identifier une pluralité de caractéristiques du signal bidimensionnel (402) comprend d'identifier une pluralité de points d'inflexion (406) du signal bidimensionnel (402).

5. Procédé selon la revendication 1, dans lequel la longueur de chaque segment (412) s'étend le long d'un second axe perpendiculaire au premier axe.

6. Procédé selon la revendication 5, comprenant en outre de détecter des maxima locaux (602) le long du premier axe et/ou du second axe pour identifier des crêtes, où, optionnellement, le signal bidimensionnel comprend un signal de PPG et où dériver des informations comprend d'analyser les crêtes détectées le long du premier axe pour calculer l'effet de phase différentielle de la respiration dans chaque segment (412).

7. Procédé selon la revendication 1, dans lequel le signal bidimensionnel comprend un signal de PPG et dans lequel dériver des informations comprend de déterminer des variations de la tension artérielle ou de déterminer un changement de la rigidité artérielle, ou de déterminer une gravité d'une maladie.

8. Système de traitement de signal comprenant :

un processeur (312) capable de :

recevoir un signal bidimensionnel (402) comprenant une composante répétitive,
identifier une caractéristique du signal bidimensionnel (402) correspondant à la composante répétitive,
transposer une pluralité de segments (412) du signal bidimensionnel pour former un empilement tridimensionnel de segments (412), les segments comprenant des points de début et des points de fin ayant généralement la caractéristique adjacente, où transposer la pluralité de segments du signal bidimensionnel comprend d'aligner chaque segment subséquent de la pluralité de segments à côté de chaque segment précédent de la pluralité de segments le long d'un premier axe ;
combiner la pluralité de segments dans l'empilement tridimensionnel de segments pour créer une surface tridimensionnelle, où la combinaison comprend d'interpoler la pluralité de segments pour créer la surface tridimensionnelle ;

**caractérisé en ce que** le processeur est capable :

d'analyser la surface tridimensionnelle pour déterminer des informations de respiration sur la base de maximas locaux de la surface.

9. Système selon la revendication 8, comprenant un capteur (12), et dans lequel le processeur (312) reçoit le signal bidimensionnel (402) du capteur (12), et
optionnellement, le capteur (12) comprend un oxymètre de pouls (320), et le signal bidimensionnel comprend un signal de photopléthysmographe (PPG).

10. Système selon la revendication 9, dans lequel dériver des informations comprend d'identifier des respirations individuelles en identifiant des maxima locaux (602) dans la surface tridimensionnelle, ou de dériver une vitesse de respiration, ou dans lequel la composante répétitive comprend une composante d'impulsion du signal de PPG.

**11.** Système selon la revendication 8, dans lequel identifier une caractéristique du signal bidimensionnel (402) comprend d'identifier un point d'inflexion (406) du signal bidimensionnel (402), et/ou le processeur (312) est capable de délivrer une alerte sur la base, au moins en partie, des informations dérivées.

**12.** Système selon la revendication 8, dans lequel la longueur de chaque segment (412) de la pluralité de segments s'étend le long d'un second axe perpendiculaire au premier axe.

**13.** Système selon la revendication 11, dans lequel le processeur (312) est capable d'identifier des crêtes dans l'empilement de la pluralité de segments, et

optionnellement, le système comprend un oxymètre de pouls (320), où le signal bidimensionnel comprend un signal de photopléthysmographe (PPG), et dériver des informations comprend d'analyser les crêtes détectées le long du premier axe pour calculer l'effet de phase différentielle de la respiration dans chaque segment (412) de la pluralité de segments.

**14.** Système selon la revendication 13, comprenant un oxymètre de pouls où le signal bidimensionnel comprend un signal de photopléthysmographe (PPG), et dériver des informations comprend de déterminer des variations de la tension artérielle ou de déterminer un changement de la rigidité artérielle, ou de déterminer une gravité d'une maladie.

**15.** Support lisible par ordinateur stockant des instructions lisibles par ordinateur qui, lorsqu'elles sont exécutées par un processeur (312), amènent le processeur (312) à exécuter un procédé selon l'une quelconque des revendications 1 à 7.

FIG.1

FIG. 2

300

310

312

Input Signal Generator

Processor

316

Oximeter

Sensor

320

318

314

Output

FIG. 3

FIG. 4

504a  504b  504c  504d  504e

**FIG. 5A**

502a  502b  502c  502d  502e

502a  502b  502c  502d

500

**FIG. 5B**

504a  504b  504c  504d  504e

504a  504b  504c  504d  504e

**FIG. 5C**

520

RECEIVE PPG
SIGNAL
STEP 522

DIGITIZE AND STORE
SIGNAL
STEP 523

DETECT TURNING
POINTS IN SIGNAL
STEP 524

APPLY
SEGMENTATION
LOGIC
STEP 526

STORE IDENTIFIED
SEGMENTS
STEP 528

TRANSPOSE
SEGMENTS TO
FORM STACK
STEP 530

DERIVE SURFACE
STEP 532

CONVERT AMPLITUDES
TO COLOR MAP
VALUES
STEP 534

FIG. 5D

FIG. 6A

EP 2 303 109 B1

FIG. 6B

**FIG. 7**

RECEIVE
SIGNAL
STEP 802

↓

IDENTIFY
REPETITIVE
FEATURES
STEP 804

↓

FORM STACK
OF SEGMENTS
STEP 806

↓

DERIVE
INFORMATION
FROM STACK
STEP 808

↓

DIAGNOSE
CONDITION
STEP 810

↓

DISPLAY
INFORMATION
STEP 812

FIG. 8

900

FIG. 9

```
        ┌─────────────┐
        │   RECEIVE   │
        │   SIGNAL    │
        │  STEP 902   │
        └──────┬──────┘
               │
               ▼
        ┌─────────────┐
        │IDENTIFY PULSES│
        │  STEP 904   │
        └──────┬──────┘
               │
               ▼
        ┌─────────────┐
        │ FORM STACK  │
        │ OF PULSES   │
        │  STEP 906   │
        └──────┬──────┘
               │
               ▼
        ┌─────────────┐
        │   IDENTIFY  │
        │   BREATHS   │
        │  STEP 908   │
        └──────┬──────┘
               │
               ▼
        ┌─────────────┐
        │  CALCULATE  │
        │  BREATHING  │
        │    RATE     │
        │  STEP 910   │
        └──────┬──────┘
               │
               ▼
        ┌─────────────┐
        │   DISPLAY   │
        │ INFORMATION │
        │  STEP 912   │
        └──────┬──────┘
               │
               ▼
        ┌─────────────┐
        │   MONITOR   │
    ┌──▶│BREATHING RATE│
    │   │  OVER TIME  │
    │   │  STEP 914   │
    │   └──────┬──────┘
    │          │
    N          ▼
    │      ╱────────╲
    │     ╱ ABNORMAL ╲        ┌─────────────┐
    └────╱BREATHING RATE?╲─Y─▶│ ISSUE ALERT │
         ╲   DB 915    ╱      │  STEP 916   │
          ╲──────────╱        └─────────────┘
```

1000

```
┌─────────────────┐
│     RECEIVE     │
│     SIGNAL      │
│   STEP 1002     │
└─────────────────┘
         │
         ▼
┌─────────────────┐
│    IDENTIFY     │
│   REPETITIVE    │
│    FEATURES     │
│   STEP 1004     │
└─────────────────┘
         │
         ▼
┌─────────────────┐
│   FORM STACK    │
│  OF SEGMENTS    │
│   STEP 1006     │
└─────────────────┘
         │
         ▼
┌─────────────────┐
│ IDENTIFY RIDGES │
│    IN STACK     │
│   STEP 1008     │
└─────────────────┘
         │
         ▼
┌─────────────────┐
│     DETECT      │
│   RESPIRATION   │
│  PHASE EFFECT   │
│   STEP 1010     │
└─────────────────┘
         │
         ▼
┌─────────────────┐
│     DETECT      │
│   COMPLIANCE    │
│    CHANGES      │
│   STEP 1012     │
└─────────────────┘
```

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61076945 A

- US 6409659 B